# EUROPEAN PATENT APPLICATION

(11) **EP 3 336 827 A1**
(43) Date of publication of application: **20.06.2018**
(21) Application number: 16834334.1
(22) Date of filing: 09.08.2016
(51) Int. Cl.: G09B 23/28

(54) **TRAINING APPARATUS FOR SURGICAL PROCEDURES AND TRAINING SYSTEM FOR LAPAROSCOPIC PROCEDURES**

(30) Priority: 11.08.2015 BR 202015019194 U; 06.04.2016 BR 102016007625
(71) Applicant: Pedroso, Miguel Angelo, 13305-430 Itu (BR); Pinho, Mauro De Souza Leite, 89203-230 Joinville (BR); Bramante, Thiago Magalhães, 18119-372 Votorantim (BR); Benevenuto, Dyego Sá, 22471-210 Rio de Janeiro (BR)
(72) Inventor: Pedroso, Miguel Angelo, 13305-430 Itu (BR); Pinho, Mauro De Souza Leite, 89203-230 Joinville (BR); Bramante, Thiago Magalhães, 18119-372 Votorantim (BR); Benevenuto, Dyego Sá, 22471-210 Rio de Janeiro (BR)
(74) Representative: Capasso, Olga
(86) International application number: PCT/BR2016/050188
(87) International publication number: WO 2017/024370

(57) **Abstract**

The present invention describes an apparatus and a system for surgical procedure training, allowing the user to simulate real surgical procedures, in addition to allowing simple assembly and disassembly thereof. Specifically, the apparatus of the present invention comprises a first region (1), a second region (2) and a third region (3) implemented in a single material, interconnected by means of points of articulation that allow folding of the material, and thereby assembling the apparatus in question. Further, a support region (4) is arranged, and it is implemented in the same material as the other regions, or individually, subsequently connected. In addition, the regions comprise fastening means for connection to one another. The present invention lies within the fields of medicine and education.

## Description

### Field of the Invention

The present invention describes an apparatus and a system for surgical procedure training, allowing the user to simulate real surgical procedures, in addition to allowing simple assembly and disassembly thereof. The present invention lies within the fields of medicine and education.

### Background of the Invention

With the advancement of medicine, several technologies regarding surgical techniques are developed in order to facilitate the work of professionals, and primarily avoiding placing the patient's life at risk. One technique applied today is called laparoscopy, which is a type of minimally invasive surgery.

Minimally invasive surgery methods are performed so that there is a small incision in the patient, where surgical devices called laparoscopes are introduced. Such devices consist of a cylindrical instrument, such as a scope, which transmits images from the patient's interior to a monitor where the surgeon can monitor the development thereof. Thus, the surgeon uses optics, tweezers, clamps, suction tubes or more instruments necessary for performing the surgical act, introducing them into the patient, and, through the video transmitted by the laparoscope, monitors the movements that are being performed inside the patient.

Therefore, such technique requires attention from the professional who performs the surgery, since there is a need for proper handling of the instruments, where a wrong movement may cause irreparable damages to the patient. To that end, some types of simulators are being developed for assisting and training these professionals who perform the surgical procedure.

However, the currently shown simulators comprise high dimensions and weight, in addition to needing fixed and specific locations for the professional to practice laparoscopic techniques. Furthermore, such simulators do not allow the professional to carry them in a simple way. Moreover, these current simulators require a set of elements and components that make up such simulators, i.e., the professional must assemble several parts in order to be able to carry out the training.

Further, different surgical procedures have different plans for incision and operation, and the existing training equipment have the limitation of having only one angle of operation, making it difficult to carry out surgical procedure training. That is, current simulators are available for a particular type of surgery, thus, if a professional needs training on different types of surgical procedures, he/she is forced to use another simulator.

While searching for the prior art in the scientific and patent literature, the following documents have been found covering with the topic:
Document US D699,297 discloses an equipment for training professionals in laparoscopic surgery comprising isolated parts being interconnected by means of hinges, so that the equipment is compact to the user. Such solution, however, comprises high manufacturing costs, since it is a rigid material with a need for hinges for interconnecting the parts. In addition, US D699,297 provides for the application of this type of simulator only for practicing a certain type of surgical procedure, thus preventing the professional from being free to practice other surgical specialties and limiting the application thereof.
Document MU 8702124 describes a simulator container for laparoscopic surgeries showing several faces with varying angles and holes for introducing the surgical elements, thus allowing the user to practice the desired procedure. Such solution, however, has a hinge in the upper region, suggesting a need to assemble different parts, making it difficult for the user to assemble it, in addition to making it difficult to transport it to other locations. Further, such solution features the use of a micro-camera, which must comprise its own lighting source, thus causing the user to be forced to acquire said object, since the container shown is isolated from external lighting. Nevertheless, the solution shown by document MU8702124 requires the user to have a specific monitor to receive the type of video signal coming from the micro-camera.
Document US 2012/082970 discloses a surgical procedure training equipment containing a top and a bottom, which are joined in order to be transported and, when the equipment is put into use, both parts are separated in the assembly thereof by means of hinges or any other element for this purpose, thus offering room for the user to practice surgical procedures. However, the solution shown in the document requires the user to assemble the parts by inserting an additional element to lock both in their respective positions, in addition to requiring the user to assemble all the apparatuses that are necessary for the training, such as, for example, the holes where the surgical elements are inserted.
Document US 6,659,776 discloses a laparoscopic procedure training apparatus comprising holes for insertion of a laparoscopic instrument and an internally coupled camera for user training video transmission. Such a solution, however, requires the user to have a monitor adapted to receive the video signal coming from the camera, in addition to not allowing several angles for training, thus precluding the professional from being free to practice other surgical specialties, and limiting the application thereof.

Thus, from what is apparent from the searched literature, no documents have been found anticipating or suggesting the teachings of the present invention, such that the solution proposed herein has novelty and an inventive step in view of the prior art.

### Summary of the Invention

The present invention has the purpose of solving the problems encountered in the prior art by developing an equipment for practicing and training surgical procedures, wherein said equipment is easy to assemble and transport, since it is implemented in continuous sheet material and comprises its regions separated by articulation points formed in the material itself. Further, the solution of the present invention comprises aperture regions to allow ambient lighting inside the equipment, allowing the user to carry out the practice without the need for additional lighting elements. In addition, the apparatus of the present invention comprises regions for training from various angles in order to enable the user to practice several different types of surgical procedures.

In a first aspect, the present invention provides a surgical procedure training apparatus comprising:
(A) a first region (1);
(B) a second region (2);
(C) a third region (3);
(D) a support region (4);
wherein,
- said apparatus is implemented in continuous sheet material comprising points of articulation between: the first region (1) and the second region (2); and the second region (2) and the third region (3);
- said points of articulation are of the same nature as the sheet material itself;
- the second region (2) comprises fastening means for connecting the support region (4);
- all said regions comprise fastening means for interconnecting one another.

In a second aspect, the present invention features a laparoscopic procedure training system comprising:
(A) a surgical procedure training apparatus;
(B) trocars;
(C) an electronic device having an image capture element;
(D) a laparoscopic instrument; and
(E) a training element;
wherein,
- said surgical procedure training apparatus is provided with apertures for introducing medical equipment and apertures for placing an image capture element;
- the laparoscopic instrument and the trocar are associated and introduced into the aperture for introducing the medical equipment of the apparatus;
- the training element is inserted inside the apparatus;
- the electronic device is associated with the apparatus so that the capture element is associated with the aperture for placing the image capture element.

Accordingly, the common inventive concept to all claimed protection contexts relates to a surgical procedure training equipment composed of at least three regions, which are separated by points of articulation of the same nature as the very material to which the regions are implemented, so that said material is flexible and allows said regions to be connected to one another by folding the material, thereby forming a type of box for surgical procedure training. In addition, said regions comprise engaging means that are adapted so that it is possible to effectuate said connections between the regions.

These and other aspects of the invention shall be immediately appreciated by persons skilled in the art and by companies with interest in the segment, and shall be described in sufficient detail for reproduction thereof in the description that follows.

### Brief Description of the Figures

With the purpose of better defining and clarifying the content of the present patent application, the following figures are shown:
- Figure 1 shows a top view of a first embodiment of the disassembled apparatus of the present invention.
- Figure 2 shows a detailed view of the first region (1) of said embodiment of the disassembled apparatus of the present invention.
- Figure 3 shows a detailed view of the second region (2) of said embodiment of the disassembled apparatus of the present invention.
- Figure 4 shows a detailed view of the third region (3) of said embodiment of the disassembled apparatus of the present invention.
- Figure 5 shows a detailed view of the support region (4) of said embodiment of the disassembled apparatus of the present invention.
- Figure 6 shows a top view of a second embodiment of the disassembled apparatus of the present invention.
- Figure 7 shows a detailed view of the first region (1) of said embodiment of the disassembled apparatus of the present invention.
- Figure 8 shows a detailed view of the second region (2) of said embodiment of the disassembled apparatus of the present invention.
- Figure 9 shows a detailed view of the third region (3) of said embodiment of the disassembled apparatus of the present invention.
- Figure 10 shows a detailed view of the support region (4) of said embodiment of the disassembled apparatus of the present invention.
- Figure 11 shows a top view of a third embodiment of the disassembled apparatus of the present invention.
- Figure 12 shows a front perspective view of the third embodiment of the assembled apparatus of the present invention.
- Figure 13 shows a rear perspective view of the third embodiment of the assembled apparatus of the present invention.
- Figure 14 shows a front view of the third embodiment of the assembled apparatus of the present invention, omitting the support region (4).
- Figure 15 shows a front view of a variation of the third embodiment of the assembled apparatus of the present invention, omitting the support region (4).

### Detailed Description of the Invention

The following descriptions are shown only by way of example and nonlimiting of the scope of the invention, and shall enable a clearer understanding of the subject of the present patent application.

In order to provide a way for users to practice surgical procedures in a single location, in addition to provide a way that is easily accessible and transported by an user, the present invention, in a first aspect, discloses a surgical procedure training apparatus comprising a first region (1), a second region (2), a third region (3) and a support region (4), such that, generally, said regions may both allow introduction of surgical elements and provide structural support for the apparatus.

The present invention apparatus can be found in at least two different manners, assembled or disassembled, so that, when disassembled, the regions are not connected to each other, where it is used in this manner to allow transportation or storage thereof; on the other hand, when shown in assembled form, the regions are engaged and the apparatus is ready to the user perform the surgical procedure training. Such functionality is provided because said apparatus is implemented in a continuous sheet material, wherein it comprises points of articulation, which are responsible for interconnecting the first region (1) to the second region (2) and the second region (2) to the third region (3). For the purposes of detailing the subject of the present invention, continuous sheet material means a single object, a single material, which may be folded, i.e., more specifically, the apparatus of the present invention is made in a single piece, requiring no external fastening elements for the interconnection of its parts.

Further, the abovementioned points of articulation are formed of the same nature as the continuous sheet material itself, i.e., are implemented within the very single piece from which the apparatus is formed. In one embodiment, the points of articulation are formed due to the fact that the material is foldable, wherein when the material is folded for assembling the apparatus, these points of articulation are formed, thus allowing full flexibility so that the regions of the apparatus are able to be connected.

In one embodiment, the continuous sheet material of the present invention is any type of material that allows folding movement, forming the points of articulation between the regions and not easily wearing out with a single assembly, so that the user may perform the assembly and disassembly steps without breakage or rupture thereof. In a more preferred embodiment, the material of the present invention is of the reinforced cardboard type, or pasteboard type.

In order to be able to implement the apparatus of the present invention in a single piece, thus allowing assembly and disassembly thereof, all regions comprise fastening means which are also formed of the same nature as the material itself. Thus, for the purposes of the present invention, said fastening means are any elements that are formed within the sheet material, and may have different geometries, such as the "male and female" type, where they are connected to each other, thus allowing assembly of the apparatus with the connection of the regions. In one embodiment, the fastening means are of the opening and projection type, wherein an opening is defined by a cut, or the removal of a small portion from part of the apparatus in question. Projections are defined as a relief, or a flap, formed by an outline during the cutting of the piece that forms the apparatus.

Further, the present apparatus comprises at least one structural support flap which is arranged in the first region (1), the second region (2) or the third region (3) by means of points of articulation. Said support flap, as well as the other elements, is also formed in the continuous sheet material itself, so as to show greater stability for assembly of the apparatus, thus enabling the user to carry out the training more steadily, similarly to what is practiced in a real surgery. In the apparatus of the present invention, said flap is implemented in one of the regions so as to connect to at least one other region, and for purposes of exemplification, the support flap is implemented in the second region (2) and connects to the third region (3), wherein this example of assembling the apparatus is not limiting, so that the support flap may be implemented in any of the regions and, therefore, connect with any of the regions.

Thus, in order to connect said support flap to the required regions, fastening means were formed in the support flap, being of the same type as the one comprised in the regions, as already defined above.

In one embodiment, the first region (1) comprises at least one aperture for introducing medical equipment, so that said aperture is disposed at a specific angle which allows the user to carry out the surgical procedure training by simulating a real surgery.

In one embodiment, the second region (2) comprises at least one aperture for introducing medical equipment, so that said aperture is disposed at a specific angle which allows the user to carry out the surgical procedure training by simulating a real surgery. Further, in said embodiment, the second region (2) comprises fastening means for connection with the support region (4), so that this connection is reinforced, thus allowing to place the necessary elements in said support region (4) without damaging the structure of the apparatus. In addition, the second region (2) comprises an aperture for image capture.

In one embodiment, the support region (4) is adapted to receive an electronic device provided with an image capture element. Furthermore, said support region (4) is provided with fastening means for connection with the second region (2), so as to comprise at least one projection perpendicular to its base, i.e., to its own construction. More specifically, said projection is disposed in the direction of the force applied by the electronic device, while said device is positioned in the support region (4) when the apparatus is assembled, wherein such arrangement is provided in order to provide greater stability and greater resistance against the weight of the electronic device when it is positioned in the support region (4), without compromising the structure of the apparatus and preventing the connection between the support region (4) and the second region (2) from yielding. In another embodiment, the support region (4) is individually implemented, not being implemented in the same continuous sheet material as the other regions; however, it has the same type of material used for the other regions. In another embodiment, the support region (4) is implemented in the same continuous sheet material as the apparatus, being interconnected to any of the regions by means of the points of articulation in the material itself.

Additionally, in one embodiment, the second region (2) comprises apertures for insertion of medical equipment at rest, i.e., when the user is not using the medical equipment for practicing the procedure, the latter may be inserted into said apertures, so as to be stored and available to the user when it is needed. Usually during a training, the user has four medical equipment, however, only two may be used at the same time by the user, thus, the apertures mentioned herein are arranged in order to allow the user to store equipment that is not in use.

The third region (3) comprised in the apparatus of the present invention may be used for various purposes and implemented in a number of different ways in order to suit the purpose for which the apparatus is used, as well as the geometry of the apparatus when assembled. In one embodiment, the third region (3) is a base region of the apparatus, serving for structural support, ensuring that the user may practice the surgical procedure more safely. In another embodiment, the third region (3) comprises sectioning and is divided into at least two portions, which are interconnected by points of articulation implemented in the sheet material itself as defined above, wherein said arrangement allows the improvement of the structural support of the apparatus. In a further embodiment, the third region (3) is divided into two parts and comprises, in one of them, at least one aperture for introducing medical equipment, so as to allow the user to have another angle to proceed with the desired surgical procedure training.

The apparatus of the present invention further comprises at least one aperture to allow the incidence of ambient lighting, wherein said aperture is implemented in any one of the regions of the apparatus, the structural support flap, or the rear of the apparatus, where the latter is formed when the apparatus is assembled, said regions having a specific geometry and, in the assembly thereof, the apparatus has a rear part with an aperture allowing the incidence of ambient lighting. Such a point is necessary to allow the user to visualize a training element when practicing the surgical procedure, without using an additional light source, or to have a light-emitting device.

In one embodiment, the apparatus of the present invention is used for laparoscopic procedure training.

In one embodiment, the aperture for introducing medical equipment is adapted for laparoscopy surgical procedure training, such as, for example, endoscopic laparoscopy, gynecological laparoscopy, transanal laparoscopy, or the like.

Thus, as may be noted, the present invention provides an easily mountable and transportable apparatus comprising various angles in its regions which allow the user to practice various types of surgical procedures in single equipment, in addition to comprising apertures to allow the incidence of ambient lighting. Further, the present apparatus is implemented entirely in foldable material, being the main part implemented in a single piece.

In a second aspect, the present invention provides a laparoscopic procedure training system comprising the above-defined apparatus, trocars, electronic device having an image capture element, laparoscopic instrument and training element. Said system is arranged to enable a user to perform laparoscopic procedure training using the apparatus of the present invention associated with the optional basic elements that enable operation thereof.

The trocar of the system, in one embodiment, is a conventional one, wherein it is an element that is inserted into a patient during a laparoscopic surgery, and the laparoscopic instruments are inserted into this element so that the surgeon may perform all the necessary steps for the procedure. In the present invention, the trocar is inserted into the aperture for surgical procedures of the apparatus in question and the laparoscopic instrument is associated with the trocar. This association allows simulating a real environment for surgery of this specialty. However, the apparatus may also be used without the use of the trocar element.

Thus, in order to proceed with the practice of the desired procedure, the user inserts a training element into the apparatus, wherein this training element is a PAD that simulates the inner tissue for practicing sutures. Thereby, the user inserts the training elements into the apparatus and starts the training.

Further, in order to simulate a laparoscopic surgery environment, the user places an electronic device in the support region (4) of the apparatus, so that the image capture element of the device is placed in the aperture for placing the image capture element comprised in the second region (2) of the apparatus. That is, accordingly, the electronic device is capable of capturing images internally to the apparatus through said aperture, where images of the training element are specifically captured and shown on the display of the device. For the purposes of the present invention, the electronic device is any device capable of capturing images and displaying them on a display, in addition to being able to be placed in the support region (4) of the apparatus, such as, for example, a tablet or an iPad®, but not limited thereto, so that the camera lens is placed in the aperture for placing the image capture element comprised in the second region (2), and the captured images from the training element are transmitted to the display of said tablet or iPad® itself.

The system of the present invention is proposed in order to show the elements that are associated with the apparatus so as to allow proper surgical procedure to be practiced, thus simulating the reality of laparoscopic surgery. However, as is apparent from its definition, the apparatus does not require these elements for its complete operation.

The examples shown herein are intended to only exemplify some of the many ways of carrying out the invention, without, however, limiting the scope thereof. Further, other embodiments of implementing the apparatus of the present invention may utilize a combination of the features shown in different examples, thus not restricting the features to the cited example.

### Example 1

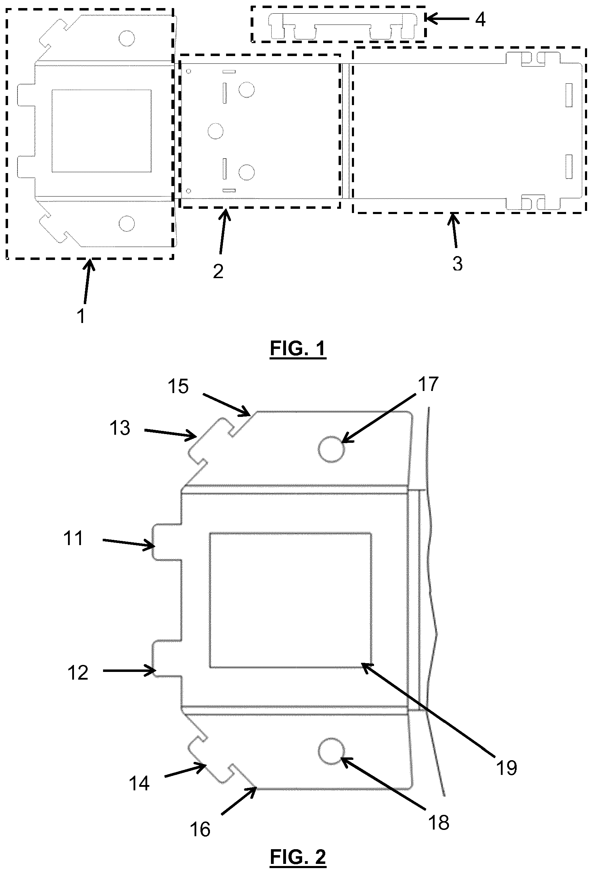

An example of the apparatus of the present invention is shown in Figures 1-5, wherein Fig. 1 shows a top view of the apparatus of the invention in a disassembled manner, wherein the first (1), second (2) and third (3) regions are separated by points of articulation of the same nature as the material itself and the support region (4) is individually implemented in additional material and, when the apparatus is assembled, said support region (4) is engaged with second region (2). In this example, the apparatus is implemented in reinforced cardboard-type material, thus allowing folding of its parts.

Fig. 2 shows a detailed view of the first region (1), showing its components, which are composed of two projections at the ends (11) and (12), a first projection (13) disposed on a first side support flap (15), a second projection (14) disposed on a second side support flap (16), side apertures (17) and (18) and an access window (19). The side aperture (17) is arranged on the first side support flap (15) and the second side aperture (18) is arranged on the second side support flap (16), wherein such side apertures allow the user to practice the surgical procedure through a side angle, thus simulating a point commonly used during real laparoscopic procedures. Further, the access window (19) is arranged in order to allow incidence of ambient lighting, so that the user may carry out the desired training.

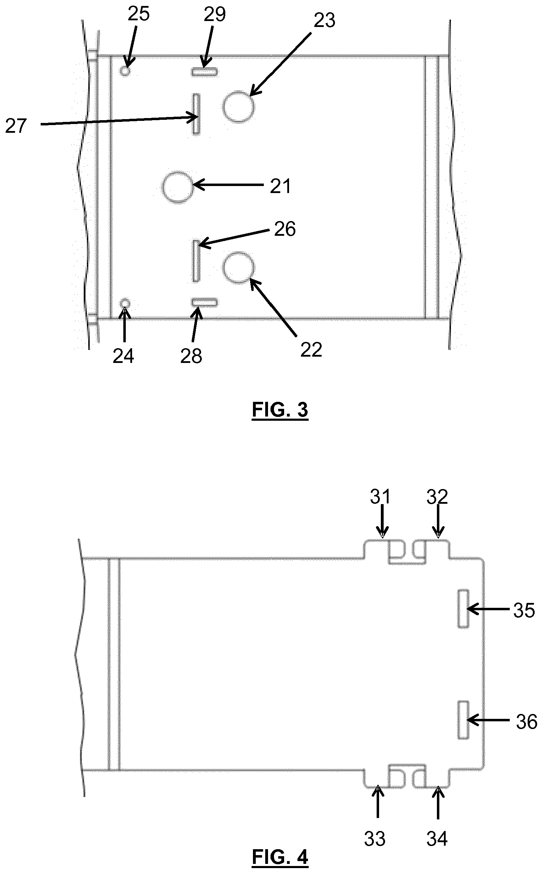

Fig. 3 shows a detailed view of the second region 2, in the present example, wherein the elements thereof are defined by an aperture for placing an image capture element (21), a first (22) and a second aperture (23) for insertion of medical equipment intended for training, a first (24) and a second aperture (25) for insertion of medical equipment at rest, i.e., equipment which are not being used, and a set of openings (26), (27), (28) and (29), intended for mounting and supporting the support region (4). The third opening (28) is perpendicular to the first opening (26) and the fourth opening (29) is perpendicular to the second opening (27), wherein said opening (29) is opposite to opening (28). Thus, as shown, the third opening (28) and the fourth opening (29) are arranged perpendicularly so as to allow the perpendicular projections of the support region (4) to be engaged.

Fig. 4 shows a detail view of the third region (3) showing its elements, namely, the side projections (31) and (32), the side projections (33) and (34) arranged opposite thereto, and the openings (35) and (36) arranged at the end.

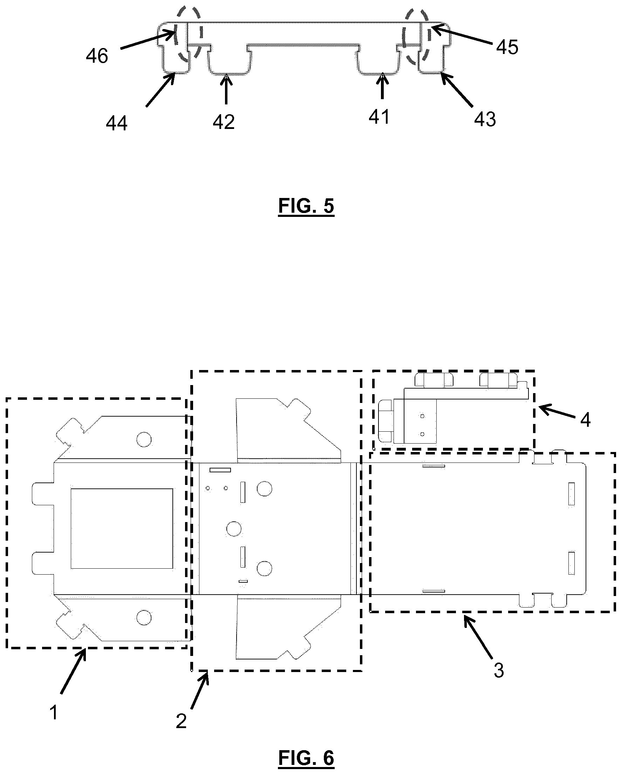

Fig. 5 shows a detail view of the support region (4) showing its components, namely, the projections (41), (42), (43) and (44). Between projections (41) and (43), and between projections (42) and (44), there is a point of articulation (45) and (46), of the same nature as the material itself, wherein folding at an angle, preferably 90°, between said projections (41) and (43) and between projections (42) and (44) is allowed. This allows for greater structural strength since, when the electronic device is placed in the support region (4), projections (43) and (44) are arranged in the same direction as the force applied by the device.

In order to assemble the apparatus, the first (1) and the third (3) regions are associated so that the projections at the ends (11) and (12) are connected to the openings (35) and (36) of the third region (3). The first projection (13) arranged on the first side support flap (15) is connected to a spacing between the side projections (31) and (32), the second projection (14), on a second side support flap (16), is connected to a spacing between the side projections (33) and (34).

Further, the support region (4) is associated with the second region (2), so that the first projection (41) of the support region (4) is introduced into the first opening (26) of the second region (2), the same occurring with the second projection (42), which is inserted into opening (27). As previously stated, the third projection (43) is folded at an angle, preferably 90°, with the first projection (41), and is thus introduced into the third opening (28), which in turn is arranged perpendicular to the first opening (26). The same also occurs with the fourth projection (44), inserted into the fourth opening (29). This fact provides a greater structural strength, which implies that the support region (4) can, thus, support greater weight.

### Example 2

Another exemplary apparatus of the present invention is shown in Figures 6-10, wherein Fig. 6 shows a top view of the apparatus of the invention in a disassembled manner, wherein the first (1), second (2) and third (3) regions are separated by points of articulation of the same nature as the material itself and the support region (4) is individually implemented in additional material and, when the apparatus is assembled, said support region (4) is engaged with second region (2). In this example, the second region (2) further comprises a support flap reinforcing the side support of the apparatus shown herein.

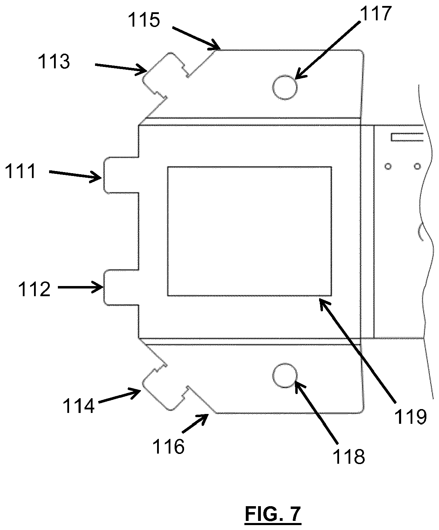

Fig. 7 shows a detailed view of the first region (1), showing its components, which are composed of two projections at the ends (111) and (112), a first projection (113) disposed on a first side support flap (115), a second projection (114) disposed on a second side support flap (116), side apertures (117) and (118) and an access window (119). The side aperture (117) is disposed on the first side support flap (115) and the second side aperture (118) is disposed on the second side support flap (116), wherein such side apertures allow the user to practice the surgical procedure through a side angle, thus simulating a point commonly used during real laparoscopic procedures. Further, the access window (119) is arranged in order to allow incidence of ambient lighting, so that the user may carry out the desired training.

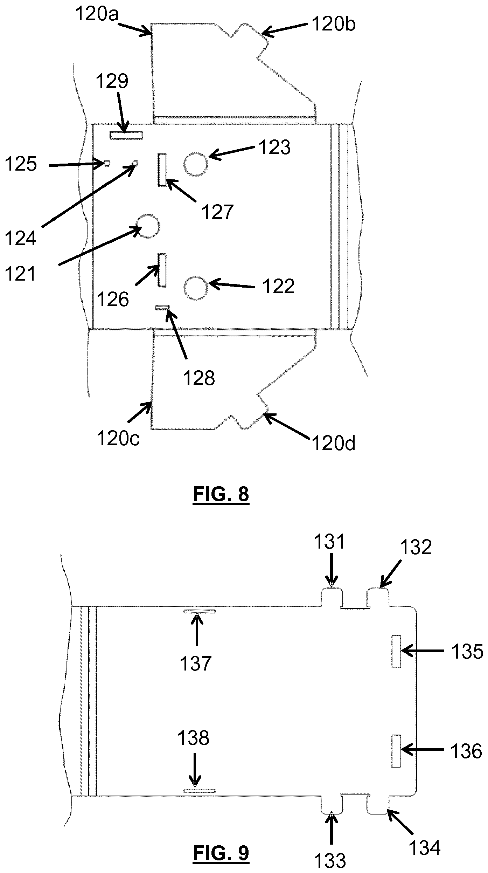

Fig. 8 shows a detailed view of the second region (2), in the present example, wherein the elements thereof are defined by an aperture for placing an image capture element (121), a first (122) and a second aperture (123) for insertion of medical equipment intended for training, a first (124) and a second aperture (125) for insertion of medical equipment at rest, i.e., equipment which are not being used, and a set of openings (126), (127), (128) and (129), intended for mounting and supporting the support region (4). The third opening (128) is perpendicular to the first opening (126) and the fourth opening (129) is perpendicular to the second opening (127), wherein said opening (129) is opposite to opening (128). Thus, as shown, the third opening (128) and the fourth opening (129) are arranged perpendicularly, wherein the fourth opening (129) is arranged displaced relative to the third opening (128), being asymmetric.

In this embodiment, the second region (2) further comprises two side support flaps, the first side support flap (120a) comprising a first projection (120b) opposite to the second side support flap (120c), which comprises a second projection (120d).

Further, Fig. 9 shows a detailed view of the third region (3) showing its components, namely, the side projections (131) and (132), the side projections (133) and (134) arranged opposite thereto, the openings (135) and (136) arranged at the end, and the openings (137) and (138) opposite to each other.

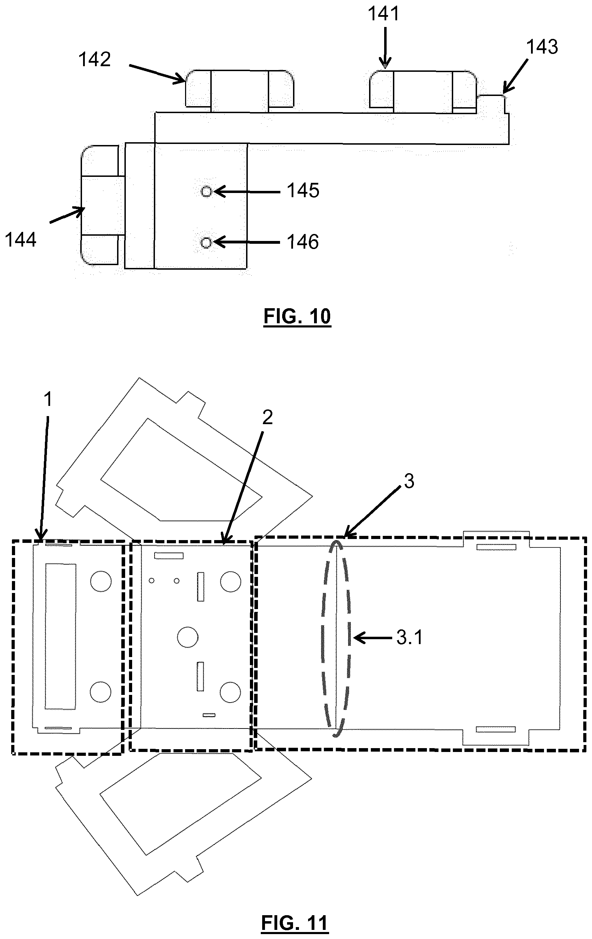

In Fig. 10, the support region (4) is shown with a detail of the parts thereof, the projections (141), (142), (143) and (144), the apertures (145) and (146) for inserting the resting surgery elements. Said apertures (145) and (146) are arranged to overlap the apertures (124) and (125) in the second region (2) at the time of assembly of the apparatus.

For assembling the apparatus, the association between the first (1) and the third (3) region is performed as shown in the abovementioned Example 1. In this example, however, the side support flaps of the second region (2) are folded at an angle of approximately 90°, and the projections (120b) and (120d) of the flaps are associated with the openings (137) and (138) of the third region (3).

### Example 3

In another exemplary apparatus of the present invention, Fig. 11 is shown, which is a top view of the apparatus of the invention in a disassembled manner, wherein the first (1), second (2) and third (3) regions are separated by points of articulation of the same nature as the material itself and the support region (4) is individually implemented in additional material and, when the apparatus is assembled, said support region (4) is engaged with second region (2). In this example, the second region (2) comprises two side support flaps.

The first region (1) comprises an aperture for incidence of ambient lighting, in addition to comprising upper apertures for introducing medical equipment, allowing training at varied angles, and also comprises openings at the side ends. The second region (2) is formed in the same manner as that shown in example 2, but the side flap, in this case, comprises a window opening so as to allow the incidence of ambient lighting, in addition to comprising top and bottom projections, wherein, when the apparatus is assembled, the top projections engage with the end openings of the first region (1) and the bottom projections engage with the end openings of the third region (3).

Further, in this example, the third region (3) comprises a sectioned region (3.1), where it is divided into two parts, so that this region is formed by points of articulation implemented in the sheet material itself. This sectioning of the third region (3) allows the increasing of the structural strength of the apparatus of the present invention.

### Example 4

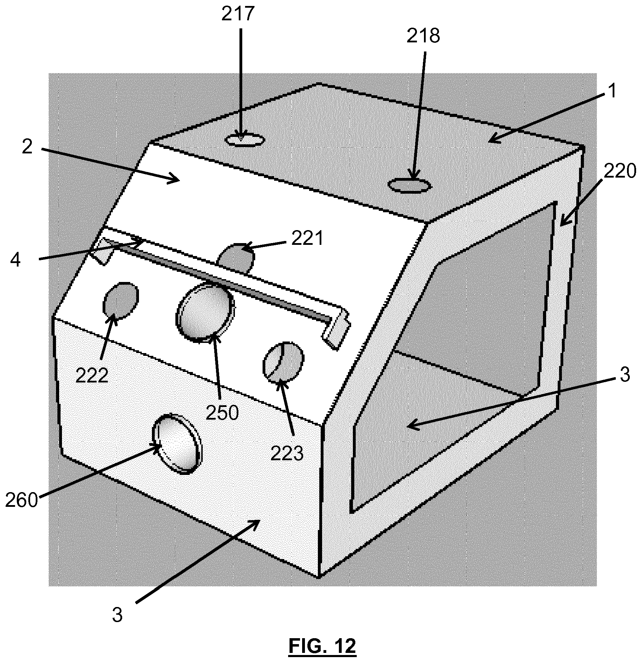

Another exemplary apparatus of the present invention is shown in figures 12 to 15, wherein Fig. 12 shows a perspective view of the apparatus of the present invention, wherein the first (1), the second (2) and the third (3) regions are separated by points of articulation of the same nature as the material itself, and the support region (4) is individually implemented in additional material and, when the apparatus is assembled, said support region (4) engages with the second region (2).

The first region (1) comprises upper apertures for introducing medical equipment (217) and (218), allowing the user to carry out the training from another angle, simulating a particular type of surgical procedure. Further, the first region (1) comprises side end openings (not shown), as shown in example 3, so as to allow connection with a side support flap (220) of the second region (2).

Accordingly, the second region (2) comprises an aperture for placing an image capture element (221), a first (222) and a second aperture (223) for introducing medical equipment, wherein said medical equipment relates to laparoscopic elements, two side support flaps (220), in addition to comprising an enlarged aperture (250) for practicing other types of surgical procedures, wherein, for example, apertures (222) and (223) are used for endoscopic laparoscopy procedures and the enlarged aperture (250) is used for performing gynecological laparoscopic procedures for transvaginal surgery.

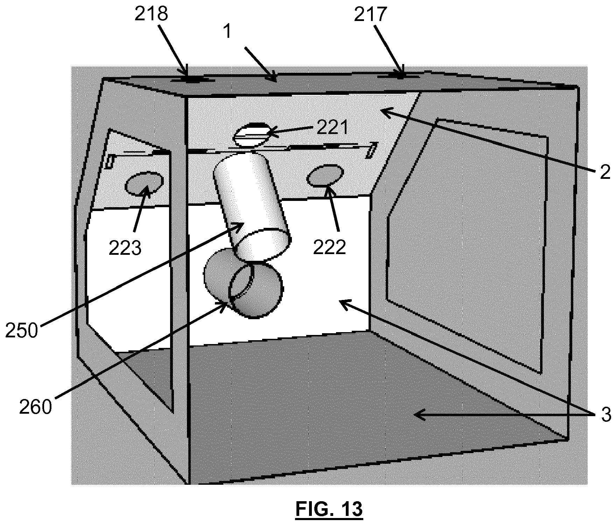

Further, in Fig. 13, where a rear perspective view of an example of the apparatus of the present invention is shown, it can be seen that the enlarged aperture (250) also comprises a cylindrical structure which enables simulating the actual transvaginal-type surgical procedure.

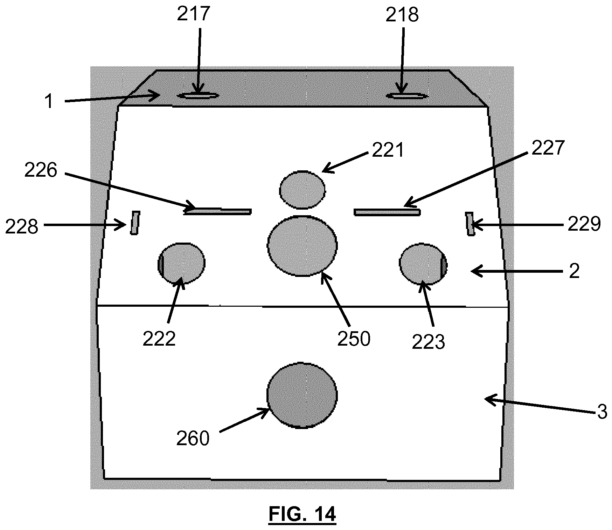

Fig. 14 shows a front view of the present example of the apparatus of the present invention, where the openings (226), (227), (228) and (229) are outlined, which enable insertion of the support region (4), so that the third (228) and the fourth (229) openings are arranged opposite to each other and perpendicular to the openings (226) and (227), whereby when the support region (4) is connected to the second region (2) the structural strength for the electronic device is improved, thus supporting greater force caused by said device.

Further, the third region (3) comprises a sectioned region as shown in Example 3 above, allowing the increasing of the structural strength of the apparatus of the present invention, where such point can be seen in Fig. 13. Notwithstanding, the third region (3) comprises an enlarged aperture (260) to allow surgical procedures to be performed, which has such angle, wherein the user inserts the laparoscopic equipment primarily perpendicularly to the third region (3) and perpendicularly to the ground or where the apparatus is positioned. Also in Fig. 13, it is shown that the enlarged aperture (260) comprises a cylindrical structure for channel simulation in a real surgical procedure, wherein this type of procedure is used in transanal laparoscopic surgery.

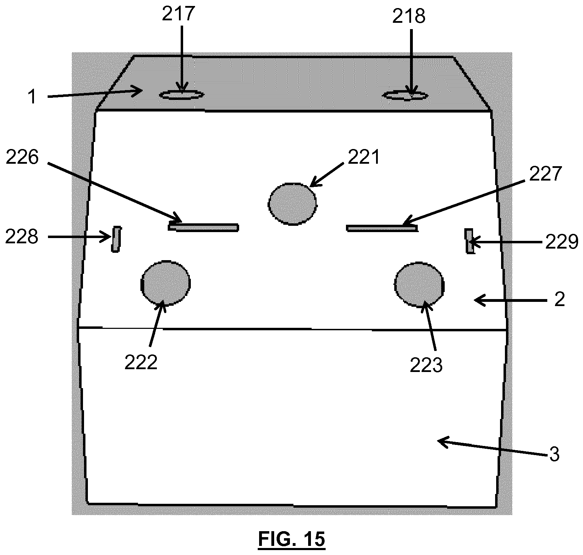

In a variation of this example, Fig. 15 shows the third region (3) without the enlarged aperture, so that said region is used only for purposes of ensuring structural strength to the apparatus of the present invention.

Those skilled in the art will appreciate the knowledge shown herein and may reproduce the invention in the modes shown and in other variants encompassed by the scope of the appended claims.

## Claims

1. A surgical procedure training apparatus **characterized in that** it comprises:
a. a first region (1);
b. a second region (2);
c. a third region (3);
d. a support region (4);
wherein,
- said apparatus is implemented in continuous sheet material comprising points of articulation between: the first region (1) and the second region (2); and the second region (2) and the third region (3);
- said points of articulation are of the same nature as the sheet material itself;
- the second region (2) comprises fastening means for connecting the support region (4);
- all said regions comprise fastening means for interconnecting one another.

2. The surgical procedure training apparatus according to claim 1 **characterized in that** it comprises at least one support flap arranged either in the first region (1), the second region (2) or the third region (3) by means of points of articulation.

3. The surgical procedure training apparatus according to claim 2 **characterized in that** the support flap comprises fastening means for connection with the other regions.

4. The surgical procedure training apparatus according to claim 1 **characterized in that** the fastening means are of the openings and projections type.

5. The surgical procedure training apparatus according to claim 1 **characterized in that** the first region (1) comprises at least one aperture for introducing medical equipment.

6. The surgical procedure training apparatus according to claim 1 **characterized in that** the second region (2) comprises at least one aperture for introducing medical equipment.

7. The surgical procedure training apparatus according to claim 6 **characterized in that** the second region (2) comprises at least one aperture for image capture.

8. The surgical procedure training apparatus according to claim 1 **characterized in that** the support region (4) is adapted to receive an electronic device having an image capture element.

9. The surgical procedure training apparatus according to claim 8 **characterized in that** the support region (4) comprises at least one perpendicular projection when attached to at least one opening in the second region (2).

10. The surgical procedure training apparatus according to any one of claims 1 to 9 **characterized in that** the second region (2) comprises apertures for inserting medical equipment at rest.

11. The surgical procedure training apparatus according to any one of claims 1 to 10 **characterized in that** the third region (3) further comprises aperture for introducing medical equipment.

12. The surgical procedure training apparatus according to any one of claims 1 to 11 **characterized in that** it comprises at least one opening for ambient lighting.

13. The surgical procedure training apparatus according to any one of claims 1 to 12 **characterized in that** the sheet material comprises sufficient flexibility to allow the process of folding.

14. The surgical procedure training apparatus according to any one of claims 1 to 13 **characterized in that** the aperture for introducing medical equipment is adapted for laparoscopy-related surgical procedure devices.

15. The surgical procedure training apparatus according to any one of claim 14 **characterized in that** the aperture for introducing medical equipment is adapted for training for surgical procedure such as endoscopic laparoscopy, gynecological laparoscopy, transanal laparoscopy, or the like.

16. Laparoscopic procedure training system **characterized in that** it comprises:
a. a surgical procedure training apparatus;
b. trocars;
c. an electronic device having an image capture element;
d. a laparoscopic instrument; and
e. a training element;
wherein,
- said surgical procedure training apparatus is provided with apertures for introducing medical equipment and apertures for placing an image capture element;
- the laparoscopic instrument and the trocar are associated and introduced into the aperture for introducing the medical equipment of the apparatus;
- the training element is inserted inside the apparatus;
- the electronic device is associated with the apparatus so that the capture element is associated with the aperture for placing the image capture element.

17. The laparoscopic procedure training system according to claim 16 **characterized in that** said surgical procedure training apparatus is as defined in claims 1 to 15.
